Europäisches Patentamt

(19) European Patent Office

Office européen des brevets

(11) Numéro de publication : **0 458 668 A1**

# (12) DEMANDE DE BREVET EUROPEEN

(21) Numéro de dépôt : **91401154.9**

(22) Date de dépôt : **30.04.91**

(51) Int. Cl.$^5$ : **C12Q 1/70,** C12Q 1/68, C07H 21/04, C07K 15/00, G01N 33/569, G01N 33/574, // C12N15/37, A61K39/21

---

Les microorganismes ont été déposés auprès de l'Institut Pasteur à Paris sous les numéros I - 756, I - 757, I - 758, I - 918.

(30) Priorité : **11.05.90 FR 9005935**

(43) Date de publication de la demande : **27.11.91 Bulletin 91/48**

(84) Etats contractants désignés :
**AT BE CH DE DK ES GB GR IT LI LU NL SE**

(71) Demandeur : **INSTITUT PASTEUR**
**25-28, rue du Docteur Roux**
**F-75724 Paris Cédex 15 (FR)**
Demandeur : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **Orth, Gérard**
**49, rue du Dr Roux**
**F-92330 Sceaux (FR)**
Inventeur : **Al-Tawheed, Anwaar**
**33, rue Charlot**
**F-75003 Paris (FR)**

(74) Mandataire : **Gutmann, Ernest et al**
**S.C. Ernest Gutmann - Yves Plasseraud 67, boulevard Haussmann**
**F-75008 Paris (FR)**

---

(54) **Sonde à papillomavirus (HPV66), notamment pour le diagnostic in vitro d'infections à papillomavirus, pouvant s'accompagner de néoplasies génitales, et produits génétiquement et immunologiquement liés à ce papillomavirus.**

(57) L'invention concerne une sonde de papillomavirus dérivés d'ADN-HPV66 déposé à la CNCM le 10 mai 1990 sous le numéro de dépôt I-951.

Cette sonde est utilisable pour la détection in vitro, par hybridation avec les ADNs contenus dans un échantillon biologique humain, de néoplasies génitales ou de cancers du col de l'utérus.

EP 0 458 668 A1

L'invention concerne un ADN de papillomavirus (HPV66) ou des variants de ce papillomavirus, et plus particulièrement des sondes dérivées de ce papillomavirus. Elle concerne aussi des produits génétiquement et immunologiquement liés à ce papillomavirus, ainsi que des procédés mettant en oeuvre ces divers produits pour le diagnostic in vitro d'infections à papillomavirus et, pour certains d'entre eux, pour la vaccination contre ces mêmes papillomavirus ou variants de papillomavirus.

Par l'expression "produit génétiquement ou immunologiquement liés à un papillomavirus", il faut entendre les divers produits dérivés de son ADN initial, qu'il s'agisse des ARN correspondants ou d'ADN recombinants contenant tout ou partie de cet ADN initial, des produits d'expression de ces ADN, le cas échéant recombinants, dans des hôtes cellulaires compétents et des anticorps dirigés contre ces produits. Il s'agit donc de polypeptides résultant de la transcription et de la traduction de tout ou partie des différentes phases de lecture ouvertes de l'ADN initial. Il s'agit encore d'anticorps induits in vivo par lesdits polypeptides.

L'expression "papillomavirus" recouvre un grand nombre de virus ayant en commun d'être tenus pour responsables de plusieurs formes d'infections virales s'étageant entre les verrues cutanées ou de muqueuses relativement bénignes et des néoplasies intra-épithéliales susceptibles de dégénérer en cancers cutanés ou muqueux. Parmi des infections à papillomavirus, on mentionnera plus particulièrement les verrues cutanées (en particulier, verrues vulgaires et plantaires), l'épidermodysplasie verruciforme, les verrues planes ou intermédiaires cutanées, les néoplasies intra-épithéliales et cancers cutanés, les cancers de l'épidermodysplasie verruciforme, les néoplasies intraépithéliales et cancers du col de l'utérus et des organes génitaux externes, les condylomes et papillomes génitaux.

Un certain nombre de types de papillomavirus a déjà été décrit. On mentionnera à titre d'exemples ceux décrits dans le brevet principal français n° 84.18369/2.578.267 et ses certificats d'addition n° 85.07073/2.581.655, le brevet n° 86.01425/2.593.828 et le brevet n° 88.08324 déposé en juin 1988, et plus récemment dans la demande de brevet n° 89 17371 déposée le 28.12.1989, tous relatifs à des types et sous-types nouveaux de papillomavirus qui ont été isolés à partir de verrues ou lésions maculaires cutanées ou de lésions génitales, parmi lesquels certains davantage susceptibles que d'autres de donner lieu au développement précoce de néoplasies génitales, notamment de cancers du col de l'utérus, chez les malades qui en sont affectés.

D'une façon générale, il a été remarqué dans les brevets antérieurs que les papillomavirus, bien que très différents entre eux, avaient des tailles de l'ordre de 7000-8000 paires de bases. En outre, leurs génomes peuvent présenter certains degrés d'homologie, évalués dans des essais d'hybridation réalisés dans des conditions dites "non stringentes" ou "non strictes" ou, au contraire, dans des conditions "d'hybridation stringente" ou "stricte".

Les essais d'hybridation dans les conditions non strictes ou non stringentes impliquent la mise en contact mutuelle d'ADN provenant de deux isolats de virus dans les conditions suivantes décrites par HEILMAN C.A. et al, 1980, J. Virol., 36, 395-407, et CROISSANT et al, 1982, C.R. Acad. Sc. Paris, 294, 581-586 (molécules hétéroduplexes).

On rappelle que ces conditions non strictes ou non stringentes impliquent la réalisation des essais d'hybridation dans les conditions, notamment de milieu et de température, suivantes :

Conditions non strictes (Tm-40°C) :

L'hybridation est effectuée à 42°C dans une solution contenant : 50 mM de tampon phosphate de sodium pH=6,5 ; 5xSSC (1xSSC=0,15 M NaCl, 0,015 M Na Citrate) ; 20 % de formamide ; 200 ug/ml d'ARN de transfert de levure ; 0,02 % de solution de Denhardt.

Les essais d'hybridation dans les conditions strictes ou stringentes impliquent la mise en contact mutuelle d'ADN provenant de deux isolats de virus dans les conditions décrites par KREMSDORF, D. ET AL. ( (1982), J. Virol. 43:436-447 et 1983, J. Virol. 48:340-351) et Davis R.W. et al., 1971, Methods Enzymol., 21, 413-418 (molécules hétéroduplexes).

On rappelle que ces conditions strictes ou stringentes impliquent la réalisation des essais d'hybridation dans les conditions, notamment de milieu et de température, suivantes :

Conditions strictes (Tm-20°C) :

L'hybridation est effectuée à 42°C dans une solution contenant : 50 mM de tampon phosphate de sodium pH=6,5 ; 5xSSC (1xxSSC = 0,15 M NaCl, 0,015 M Na Citrate) ; 50 % de formamide ; 200 ug/ml d'ARN de transfert de levure ; 0,02% de solution de Denhardt.

Il a été dit dans les brevets antérieurs que les papillomavirus qui présentent des pourcentages d'hybridation croisée inférieurs à 50 % dans des conditions strictes ou stringentes appartenaient à des types différents. Les virus pour lesquels on observe, dans ces conditions strictes ou stringentes, des pourcentages (ou degrés) d'hybridation supérieurs à 50 % sont considérés comme appartenant au même type. Ils peuvent former des sous-types différents au sein de ce même type. Pour mémoire, on rappellera que deux HPV appartiennent à des types différents si leurs génomes présentent moins de 50 % d'hybridation croisée dans des conditions strictes d'hybridation, c'est-à-dire après hybridation en milieu liquide à saturation et traitement des hybrides par la nucléase S1. Deux HPV représentent des sous-types du même type si leurs génomes présentent une homologie incomplète, mais supérieure à 50 %. Des différences ne portant que sur un petit nombre de sites de coupure par des enzymes de restriction définissent des variants.

Les brevets antérieurs ont également décrit l'utilisation des ADNs dérivés de ces papillomavirus, en particulier de recombinants génétiques comprenant tout ou partie des génomes (dénommés ADN-HPVs) de ces papillomavirus, en tant que sondes d'hybridation. L'utilisation de mélanges ou "cocktails" d'ADN-HPVs, pour la préparation de mélanges de sondes d'hybridation, a également été décrite. Ces mélanges de sondes sont alors susceptibles d'être mis en oeuvre, quelquefois plus efficacement que des sondes isolées, pour le diagnostic in vitro de diverses catégories d'infections, voire des niveaux de risque qui accompagnent la découverte chez un patient de papillomavirus déterminés. Au titre de ces catégories d'infections, on citera celles qui sont susceptibles de s'accompagner de néoplasies génitales et, en particulier, de cancers de l'utérus : voir par exemple les cocktails indiqués plus loin (si l'on fait abstraction de l'ADN-HPV qui est l'objet de la présente invention).

Pour mémoire, on citera, parmi les sondes susceptibles d'être mises en oeuvre dans les essais de diagnostic in vitro susmentionnés, celles qui peuvent être constituées à partir de sondes HPV54, HPV55 (a et b), décrites dans le brevet n° 88.08324 et HPV63 décrite dans la demande de brevet 89 19371, dont les cartes de restriction ont été reproduites dans les figures 1, 2 et 3 des dessins ci-annexés.

L'invention concerne un papillomavirus nouvellement isolé, l'ADN génomique qui peut être obtenu à partir de ce nouveau papillomavirus, des fragments de cet ADN génomique, ainsi que les nouvelles sondes d'hybridation qui peuvent être formées à partir de cet ADN-HPV ou de ces fragments d'ADN-HPV.

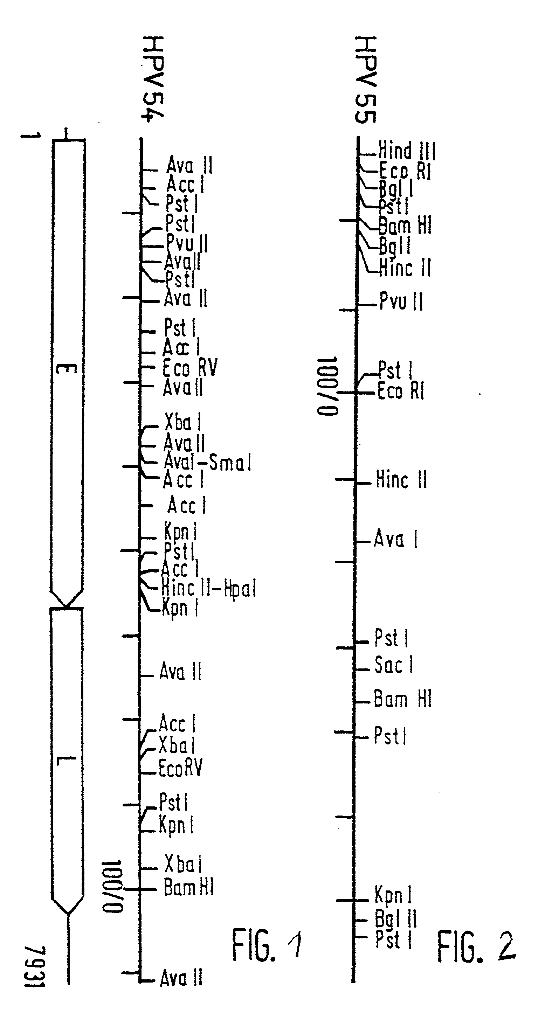

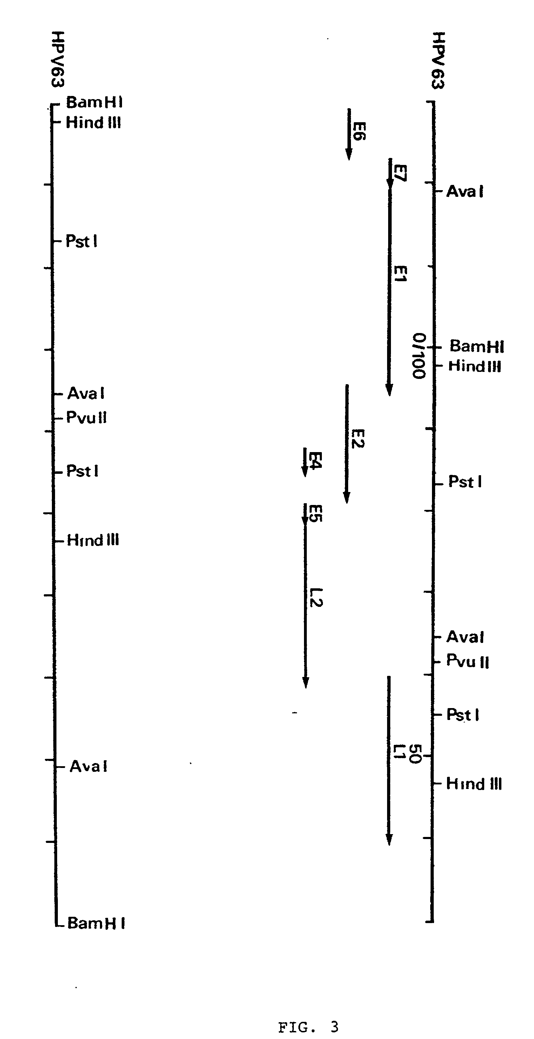

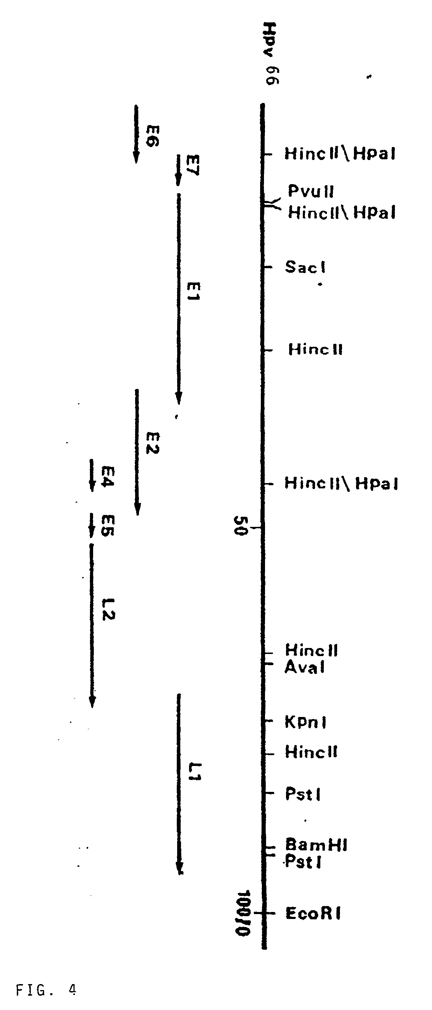

L'ADN de ce nouvel HPV (ci-après dénommé HPV66), qui a été isolé à plusieurs reprises, à partir de lésions présentant les caractéristiques cliniques des néoplasies intra-épithéliales génitales du col de l'utérus, est caractérisé par la carte de restriction faisant l'objet de la figure 4 jointe.

La carte physique donne la position de sites de coupure par diverses endonucléases de restriction. L'origine de la carte est constituée par un site unique de coupure, en l'occurrence un site EcoRI. Les distances des autres sites par rapport à l'origine sont exprimées en pourcentage de longueur de génome.

La découverte de ce nouveau papillomavirus et la production des ADN qui peuvent en être dérivés fournit donc la possibilité de réaliser des diagnostics plus affinés, notamment des infections à l'origine de lésions génitales imputables à des papillomavirus ou susceptibles de se développer sous l'effet de ces derniers, et de mieux pronostiquer le degré de risque que ces infections se transforment en des maladies plus redoutables.

L'invention concerne également tous les variants de ces papillomavirus, appartenant respectivement aux mêmes sous-types, donc capables de s'hybrider avec l'ADN-HPV66 dans des conditions stringentes.

L'invention concerne encore des "cocktails" de sondes déjà décrits dans les brevets antérieurs, mais cependant complétés avec une sonde dérivée du papillomavirus selon la présente demande et, par conséquent, des trousses ou "kits" de diagnostic encore davantage perfectionnés.

Les sondes d'hybridation construites à partir du génome de HPV66 sont particulièrement utiles, pour le diagnostic et/ou le dépistage in vitro, dans les conditions qui ont été décrites dans les brevets antérieurs et qui seront encore rappelées plus loin, des types de papillomavirus qui constituent un risque, notamment de néoplasies génitales et cancers du col de l'utérus.

Ces sondes d'hybridation sont avantageusement incorporées à des "cocktails" de diagnostic des affections du même type. Il est encore fait référence aux brevets antérieurs dans lesquels figurent les cartes de restriction des constituants autres que HPV54, et 55 de ces mélanges de diagnostic. A ce titre, les brevets antérieurs doivent être considérés comme faisant partie de la présente description, pour ce qui est de l'identification des autres ADN-HPVs entrant dans la composition de ces "cocktails".

L'invention concerne également des fragments de l'ADN-HPV66 ou des fragments d'ADN, en particulier de longueurs équivalentes, capables de s'hybrider avec le précédent, notamment dans des conditions strictes. De même, elle concerne les ADN recombinants contenant tout ou partie des ADN-HPV sus-indiqués, et plus particulièrement des ADN recombinants contenant, respectivement, des fragments correspondant aux gènes E1, E2, E4, E5, E6-E7, L1, L2 et à la région intergénique, NC, ou encore des fragments contenant des séquences correspondant aux régions intergéniques de chacun desdits ADN-HPV. Elle concerne aussi les sondes qui peuvent être constituées à partir de ces ADN-HPV ou à partir de tout fragment contenu dans cet ADN-HPV, dès lors qu'il est capable de s'hybrider avec le précédent dans des conditions strictes et dans des conditions non strictes avec les ADN-HPV correspondant aux types antérieurement décrits HPV16, HPV18 et HPV33 et les procédés de diagnostic in vitro mettant en jeu lesdites sondes ou les mélanges les contenant.

Clonage moléculaire et caractérisation d'un nouveau type d'HPV associé à des néoplasies génitales (HPV66) :

Le nouveau type d'HPV a été mis en évidence dans l'ADN extrait d'un cancer épidermoide du col utérin, par hybridation dans des conditions non strictes (Tm-40°C), à l'aide d'un mélange de sondes radioactives spécifiques de l'ADN des HPV16, 18 et 33. Une étude de la sensibilité à plusieurs endonucléases de restriction a montré que l'ADN du nouvel HPV était coupé une fois par l'endonucléase EcoRI, engendrant des molécules d'environ 8 kilobases (taille moyenne des génomes de HPV). Après digestion de la préparation d'ADN tumoral par l'enzyme EcoRI, les fragments d'ADN ont été insérés dans l'ADN du bactériophage lambda Zap. Après encapsidation de l'ADN recombinant et infection de bactéries Escherichia coli souche XLI-Blue, les bactériophages recombinants ayant inséré l'ADN du nouvel HPV ont été sélectionnés par la technique d'hybridation sur plages de lyse (Benton et Davis), en utilisant une sonde d'ADN spécifique des HPV16, 18 et 33 dans des conditions non strictes d'hybridation. Plusieurs recombinants contenant la totalité des séquences virales ont été isolés. Le traitement de l'ADN des bactériophages recombinants et de la préparation d'ADN extraite du cancer invasif par le mélange des endonucléases EcoRI et PstI, engendre les mêmes fragments, dont le poids moléculaire total correspond à celui d'un génome d'HPV. L'ADN du nouvel HPV a été excisé des séquences d'ADN de phage et recloné dans le plasmide pBR322.

Une carte de restriction de l'ADN du nouvel HPV (fig. 4) a été construite à partir de l'étude de la sensibilité de cet ADN à 15 endonucléases de restriction. 17 sites de coupure ont été localisés. Cette carte est différente de celle de tous les HPV caractérisés à ce jour. Le degré d'homologie entre l'ADN du nouvel HPV et l'ADN des HPV connus a été analysé par des expériences d'hybridation sur réplique effectuées dans des conditions très strictes (Tm-10°C), en utilisant une sonde d'ADN radioactif spécifique du nouvel HPV. Une hybridation partielle a été observée avec l'ADN des HPV53 et 56. Le degré d'homologie entre l'ADN du nouvel HPV et l'ADN des HPV53 et 56 est inférieur à 50% par des expériences d'hybridation en milieu liquide à saturation, suivies par une digestion par la nucléase S1. Le virus isolé d'un cancer invasif du col utérin constitue donc un nouveau type de HPV, dénommé provisoirement HPV66.

L'analyse, au microscope électronique, de molécules hétéroduplexes formées entre l'ADN du HPV66 et l'ADN du HPV16 et l'établissement de la séquence nucléotidique d'un fragment d'ADN du HPV66 localisé dans la phase ouverte de lecture (POL) E6 a permis d'aligner la carte physique de l'ADN du HPV66 avec la carte des POL du génome du HPV16. Dans le tableau qui suit sont rapportées les localisations putatives des principaux gènes et de la région intergénique de régulation du HPV66 sur la carte de ce génome.

## Coordonnées des extrémités (%)

|  | | 5' | 3' |
|---|---|---|---|
| | .......................... | | |
| E6 | .......................... | 5 | 11,2 |
| E7 | .......................... | 11 | 15 |
| E1 | .......................... | 15 | 30 ,8 |
| E2 | .......................... | 38,7 | 53 |
| E4 | .......................... | 46,4 | 50 |
| E5 | .......................... | 53 | 56 |
| L2 | .......................... | 56,5 | 75,7 |
| L1 | .......................... | 74 | 94,7 |
| Région de régulation | ......... | 94,7 | 5 |

D'une façon générale, l'invention concerne donc également tout ADN recombinant contenant l'ADN-HPV susdit ou des fragments de cet ADN-HPV, en particulier des sondes d'hybridation formées de ces ADNs recombinants et spécialement adaptées à la détection d'une infection par le papillomavirus selon l'invention ou d'un variant ou sous-type de ce papillomavirus. Ces sondes peuvent, soit être marquées elles-mêmes, soit être modifiées au niveau de certains nucléotides, notamment en vue de leur couplage, direct ou indirect, avec un marqueur distinct. Il va de soi que dans ces sondes les parties étrangères à la séquence nucléotidique corres-

EP 0 458 668 A1

pondante à l'ADN du papillomavirus et provenant normalement d'un vecteur de clonage, sont telles qu'elles ne risquent pas d'hybrider dans des conditions stringentes avec les autres acides nucléiques éventuellement contenus dans l'échantillon testé pour sa teneur éventuelle en ADN du papillomavirus correspondant ou de l'un de ses variants.

Le procédé selon l'invention pour le diagnostic in vitro sur un échantillon biologique à tester, provenant normalement d'un patient humain, d'une infection par un papillomavirus pouvant entraîner ou ayant entraîné une néoplasie génitale et/ou un cancer du col de l'utérus, par exemple un cancer épidermoïde du col utérin ou encore les lésions du type néoplasie intraépithéliale du col utérin ou du pénis est donc caractérisé par la mise en contact d'une sonde telle que ci-dessus définie avec les acides nucléiques de cet échantillon, le cas échéant préalablement rendus accessibles à la sonde, de préférence dans des conditions d'hybridation stringentes, et par la détection de l'hybride formé entre l'ADN viral recherché, éventuellement présent dans l'échantillon et ladite sonde.

L'utilisation de sondes radioactives préparées à partir de l'ADN du HPV66 purifié a permis de déterminer le pouvoir pathogène de ce virus. La recherche de l'ADN du HPV66 dans 160 préparations d'ADN extraites de lésions génitales contenant des génomes d'HPV différents de ceux identifiés à ce jour, a permis de révéler la présence de l'ADN du HPV66 dans trois néoplasies intraépithéliales du col de l'utérus (6,4 %) et une lésion du pénis. HPV66 constitue donc un type d'HPV à tropisme génital présentant un potentiel oncogène. Il est très souhaitable de l'incorporer à tout mélange d'ADN d'HPV destiné à la préparation de sondes moléculaires, en vue du diagnostic ou du dépistage des types d'HPV constituant un risque pour le développement de néoplasies génitales et, en particulier, de cancers du col de l'utérus.

Dans une variante de l'invention, la sonde est donc associée à des sondes dérivées de un ou plusieurs autres papillomavirus, en particulier de ceux désignés ci-après :

– HPV16, 18, 33, 39, 54, 63 ou en variante encore, avec les HPV6, 11, 42, 55, pour le diagnostic in vitro de néoplasies génitales et cancers du col de l'utérus, ou de condylomes et papillomes.

Chacune des sondes selon l'invention ou les mélanges contenant la susdite sonde peuvent notamment être utilisés comme suit, étant naturellement entendu que les essais de diagnostic décrits ne sauraient être considérés comme limitatifs des conditions d'emploi des sondes ou mélanges de sondes selon l'invention.

Dans l'exemple considéré, il s'agit par exemple d'identifier un HPV dans une biopsie, dans des cellules obtenues par grattage de lésions, ou dans des coupes de biopsies fixées par le mélange de Carnoy (éthanol, chloroforme, acide acétique 6:3:1) et incluses dans la paraffine. L'Examen nécessite l'extraction préalable de l'ADN des prélèvements selon des méthodes dont le principe est connu et met en jeu l'analyse de cet ADN par des expériences d'hybridation moléculaire, effectuées dans des conditions strictes ou moins strictes, à l'aide de sondes radioactives (marquées au $^{32}$ p ou au $^{35}$ S) préparées à partir de l'HPV selon l'invention ou de mélanges d'ADN d'HPV le contenant.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou "Genescreenplus"), l'hybridation de chaque membrane, dans les conditions usuelles, avec un mélange de sondes et la détection des hybrides radioactifs, par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, "Genescreenplus") et leur hybridation, dans les conditions usuelles, avec le mélange approprié de sondes. La formation d'hybrides radioactifs est détectée après exposition des membranes au contact d'un film radiographique.

Les sondes radioactives sont constituées soit par des ADN d'HPV marqués par la méthode de translation de coupure ("nick-translation"), soit par des ARN préparés par transcription d'ADN viraux insérés dans un vecteur, par exemple de type SP6. L'utilisation de sondes radioactives présente l'avantage d'une grande sensibilité, mais ceci n'exclut pas l'utilisation de sondes non radioactives, par exemple de sondes biotinylées et susceptibles d'être reconnues par des anticorps soit marqués eux-mêmes, soit eux-mêmes reconnus par des anticorps portant un marqueur enzymatique, fluorescent, etc...

L'invention concerne également des cultures cellulaires compétentes transformées avec des ADNs recombinants du type sus-indiqué, en particulier ceux dans lesquels la séquence nucléotidique correspondant à l'ADN du papillomavirus est placée sous le contrôle d'éléments de transcription et de régulation de cette séquence nucléotidique dans ladite culture cellulaire.

Par conséquent, elle concerne également les produits d'expression de ces ADNs recombinants dans les hôtes cellulaires compétents correspondants et les anticorps correspondants susceptibles d'être produits contre ces produits d'expression.

A ce titre, l'invention concerne les polypeptides résultant notamment des expressions respectives des

5

gènes E1, E2, E4, E6, E7, L1, L2 de l'ADN-HPV66.

Le procédé selon l'invention pour la production de ces polypeptides comprend par conséquent la transformation de cultures cellulaires compétentes avec les ADNs recombinants contenant les séquences nucléotidiques correspondantes issues de HPV66, de façon telle que la séquence nucléotidique correspondant à l'une desdites protéines puisse être exprimée dans cet hôte cellulaire, la récupération de ces polypeptides à partir des produits synthétisés par l'hôte cellulaire compétent et la purification (par exemple par mise en contact des produits d'expression préalablement extraits des cultures cellulaires ou du milieu dans lequel celles-ci ont été développées, avec des anticorps préalablement formés contre de tels polypeptides).

Les produits d'expression des séquences L2 de chacun des génomes des papillomavirus selon l'invention présentent cependant un intérêt tout particulier, en ce qu'ils peuvent eux-mêmes être utilisés pour la production in vivo d'anticorps susceptibles de reconnaître les produits d'expression du gène L2 dans des prélèvements biologiques affectés par un papillomavirus du type HPV66 ou d'un variant de celui-ci, et ce plus particulièrement lorsque les préparations du genre en question ont préalablement été fixées.

L'invention concerne encore des polypeptides hybrides contenant les polypeptides susdits et dérivés respectivement de HPV66, par exemple la protéine L2 fusionnée à d'autres séquences polypeptidiques, dans la mesure où celles-ci ne modifient pas de façon essentielle les propriétés immunogènes de la protéine L2. La présence de ces autres fragments polypeptidiques peut notamment résulter du mode de production utilisé de ces polypeptides hybrides, par exemple par génie génétique. Par exemple, ces polypeptides hybrides contiennent une séquence dérivée de la β-galactosidase. De tels produits peuvent notamment être obtenus par transformation de E. coli avec des vecteurs appropriés (phages ou plasmides) modifiés par tout ou partie de l'opéron lactose et comportant en outre, insérée en aval du promoteur de l'opéron-lactose (ou de tout autre promoteur approprié, par exemple de phage λ), la séquence nucléotidique dérivée d'un gène L2 issu du papillomavirus concerné selon l'invention. On a avantageusement recours à des plasmides ou phages de ce type comprenant une partie au moins du gène de la β-galactosidase de l'opéron-lactose.

Les polypeptides selon l'invention peuvent également, lorsqu'ils ont été purifiés, être mis en oeuvre dans des techniques de purification des anticorps qui leur correspondent, notamment à partir de sérums d'animaux qui avaient été immunisés par ces polypeptides. En particulier, ces polypeptides peuvent ête fixés sur des colonnes d'affinité. Les opérations de purification des anticorps consistent alors à faire passer le sérum les contenant au contact de colonnes d'affinité portant les susdits polypeptides. Les anticorps sélectivement fixés sur ces colonnes peuvent ensuite être récupérés par dissociation des complexes antigènes-anticorps, au moyen d'un tampon approprié, présentant une force ionique adéquate, par exemple une solution aqueuse d'un sel tel que l'acétate d'ammonium. On peut également avoir recours à des solutions acidifiées.

L'invention concerne également un procédé de production d'anticorps contre lesdits polypeptides, en particulier contre les produits d'expression des gènes E6, E7 ou, de préférence L2 de HPV66, ce procédé comprenant l'immunisation d'un hôte vivant approprié avec lesdits polypeptides et la récupération des anticorps formés à partir d'un sérum de l'hôte immunisé, notamment par mise en contact de ces sérums avec les polypeptides correspondant à l'état purifié et par la récupération de ces anticorps à partir des complexes antigène-anticorps formés.

L'invention concerne enfin les compositions mettant en jeu les anticorps selon l'invention (ou groupes contenant ces anticorps en association avec des anticorps issus d'autres papillomavirus), en particulier les compositions contenant les anticorps dérivés des compositions ou "cocktails" d'ADN-HPVs répertoriés plus haut et sur lesquels il est encore revenu dans ce qui suit (ou groupes d'anticorps correspondants).

En particulier, l'invention concerne les anticorps ou mélanges d'anticorps susdits, préalablement purifiés, en association avec un véhicule pharmaceutique approprié. Cette composition est alors susceptible d'être mise en oeuvre pour le traitement de l'affection donnée, dès lors que celle-ci aurait été diagnostiquée cliniquement, à l'issue d'un essai de diagnostic in vitro sur un prélèvement histologique ou cytologique provenant du malade. Cette composition (notamment sous forme de sérum) peut être administrée, de préférence par voie parentérale. Ce sérum est alors susceptible de provoquer une régression des infections induites par des papillomavirus du type HPV66.

Ces anticorps peuvent plus particulièrement être mis en oeuvre dans des essais de diagnostic d'une infection à l'égard de l'un des papillomavirus selon l'invention ou de variants de ces papillomavirus, dans la mesure où des coupes histologiques provenant des personnes infectées peuvent également contenir des produits d'expression de certains des gènes de structure, notamment L2, de papillomavirus du même type.

L'invention concerne donc encore un procédé de diagnostic in vitro, en particulier de néoplasies génitales et de cancers du col de l'utérus, comprenant la mise en contact de coupes histopathologiques provenant de lésions induites chez les personnes concernées dans des conditions permettant la production d'un complexe antigène-anticorps et la détection de ce complexe antigène-anticorps. Avantageusement, la détection se fait sur des préparations fixées au préalable dans des conditions dissociantes, par exemple avec le milieu ou

6

mélange de CARNOY déjà mentionné plus haut (également décrit dans l'ouvrage de L. LISON, intitulé "Histochimie et cytochimie animales").

Les anticorps anti-L2 éventuellement fixés peuvent être reconnus par d'autres anticorps formés contre les premiers, ces autres anticorps portant des marqueurs appropriés, de préférence non radioactifs. Ces marqueurs sont par exemple de nature enzymatique ou fluorescente.

Les anticorps ainsi sélectionnés, comme les sondes d'hybridation sus-définies qui leur correspondent, peuvent par conséquent être utilisées pour diagnostiquer in vitro les types d'affections correspondants.

L'invention concerne enfin les compositions vaccinantes correspondantes contenant une ou de préférence plusieurs autres protéines L2, en association avec un véhicule pharmaceutiquement acceptable adapté au mode d'administration choisi, notamment par voie parentérale. Ces compositions sont utilisables pour protéger les personnes soumises à de hauts risques d'infection par HPV66 ou par des papillomavirus de type correspondant.

La souche contenant l'ADN-HPV66 a été déposée à la CNCM le 10 mai 1990 sous le numéro de dépôt 1-951 La souche déposée consiste en une culture de E. coli K12 EcoRI souche C 600. hébergeant un plasmide recombinant EcoRI contenant lui-même l'ADN de HPV66.

Il est rappelé que les HPV54, 55A, 55B et 63 ont été déposés à la CNCM (sous les formes aussi indiquées ci-dessous) le 6 mai 1988, sous les numéros suivants :

```
- PSP 64 HPV54                    : I-756
- PGM  4 HPV55A                   : I-757
- PGM  4 HPV55B                   : I-758
  et
- Souche NM522 contenant l'ADN-HPV63 : I-918   déposée le
  21 décembre 1989
```

## Revendications

1 - ADN de papillomavirus caractérisé en ce qu'il consiste essentiellement en l'ADN caractérisé par la carte de restriction de la figure 4 (HPV66) ou en un fragment de celui-ci, ou encore en un ADN ou fragment d'ADN correspondant hybridant avec les précédents dans des conditions strictes.

2 - ADN de papillomavirus selon la revendication 1, caractérisé en ce qu'il correspond à celui contenu dans le dépôt effectué auprès de la CNCM le 10 mai 1990 sous le n° I-951 ou à un fragment de celui-ci, ou encore en un ADN ou fragment d'ADN correspondant hybridant avec les précédents dans des conditions strictes.

3 - ADN selon la revendication 1 ou la revendication 2, caractérisé en ce qu'il consiste en un fragment hybridant dans des conditions strictes avec HPV66 et dans des conditions non strictes avec HPV16, HPV18 ou HPV33.

4 - ADN selon la revendication 3, caractérisé en ce que ledit fragment correspond à l'un des gènes suivants de HPV66 : E1, E2, E4, E5, E6-E7, L1, L2 ou à tout ou partie de la région intergénique NC.

5 - Sonde d'hybridation constituée par un ADN dérivé de l'ADN selon l'une quelconque des revendications 1 à 4.

6 - Polypeptide correspondant au produit d'expression de l'un des gènes de structure de l'un des ADNs de papillomavirus, selon l'une quelconque des revendications 1 à 4.

7 - Polypeptide selon la revendication 6, caractérisé en ce qu'il consiste plus particulièrement en un produit d'expression de la séquence L2 du génome du papillomavirus HPV66.

8 - Anticorps dirigés contre le polypeptide selon la revendication 6 ou la revendication 7.

9 - Procédé de détection in vitro sur un échantillon biologique à tester, d'une infection par un papillomavirus du type HPV66, caractérisé par la mise en contact d'une sonde correspondante, telle que définie dans la revendication 5, avec les acides nucléiques de cet échantillon, le cas échéant préalablement rendus accessibles à la sonde, de préférence dans des conditions d'hybridation stringente, et par la détection de l'hybride formé entre l'ADN viral recherché, éventuellement présent dans l'échantillon, et ladite sonde.

10 - Procédé selon la revendication 9, caractérisé en ce que le diagnostic in vitro est orienté vers la détection de néoplasies génitales, et de cancers du col de l'utérus ou du pénis.

11 - Application des anticorps selon la revendication 8 au diagnostic in vitro d'une infection par un papillomavirus d'un type susceptible d'induire des néoplasies génitales et des cancers du col de l'utérus, caractérisée par la mise en contact d'un anticorps, tel que défini dans la revendication 8, avec un échantillon biologique provenant de la personne soumise au diagnostic in vitro, et par la détection du complexe antigène-anticorps formé.

12 - Mélange d'ADNs de papillomavirus comprenant un ADN conforme à l'une quelconque des revendications 1 à 4 ou une sonde conforme à la revendication 5, associé à un ou plusieurs des ADNs correspondants de papillomavirus distincts, notamment des suivants, ou aux mélanges qui apparaissent successivement aux lignes suivantes :
- HPVs 16, 18, 33, 39, 54
- HPVs 6, 11, 42, 54
- HPVs 6, 11, 42, 55,
- HPVs 6, 11, 42, 54 et 55.

13 - Mélange de polypeptides correspondant aux produits d'expression de gènes de structure des ADNs HPV de la revendication 12.

14 - Mélange d'anticorps dirigés contre les polypeptides selon la revendication 13.

EP 0 458 668 A1

FIG. 2

FIG. 1

FIG. 3

FIG. 4

Office européen
des brevets

**RAPPORT DE RECHERCHE EUROPEENNE**

Numero de la demande

EP 91 40 1154

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | WO-A-8 701 375 (INSTITUT PASTEUR) <br> * Abrégé; revendications 1-8 * <br> --- | 5-14 | C 12 Q 1/70 <br> C 12 Q 1/68 <br> C 07 H 21/04 <br> C 07 K 15/00 <br> G 01 N 33/569 <br> G 01 N 33/574 // <br> C 12 N 15/37 <br> A 61 K 39/21 |
| D,A | EP-A-0 192 001 (INSTITUT PASTEUR) <br> * Abrégé; revendications 1-9 * <br> --- | 5-14 | |
| A | WO-A-8 909 940 (ONCOR INC.) <br> * Revendications 1-47,64-71 * <br> --- | 5-14 | |
| D,A | EP-A-0 342 128 (INSTITUT PASTEUR) <br> * En entier * <br> --- | 5-14 | |
| D,A | EP-A-0 235 004 (INSTITUT PASTEUR) <br> * Abrégé; revendications 1-9 * <br> ----- | 5-14 | |

DOMAINES TECHNIQUES RECHERCHES (Int. Cl.5)

C 12 Q
C 12 N
A 61 K
C 07 K
C 12 P

Le présent rapport a été établi pour toutes les revendications

| Lieu de la recherche | Date d'achèvement de la recherche | Examinateur |
|---|---|---|
| LA HAYE | 04-09-1991 | OSBORNE H.H. |